# EUROPEAN PATENT SPECIFICATION

(11) **EP 3 900 764 B1**
(45) Date of publication and mention of the grant of the patent: **09.07.2025**
(21) Application number: 20315203.8
(22) Date of filing: 21.04.2020
(51) Int. Cl.: A61M 5/34, A61M 39/10

(54) **A MEDICAL CONTAINER, AN ADAPTOR FOR MOUNTING ONTO SAID MEDICAL CONTAINER, AND A DRUG DELIVERY DEVICE COMPRISING SAID MEDICAL CONTAINER**
MEDIZINISCHER BEHÄLTER, ADAPTER ZUR BEFESTIGUNG AUF BESAGTEM MEDIZINISCHEN BEHÄLTER UND ARZNEIMITTELABGABEVORRICHTUNG MIT BESAGTEM MEDIZINISCHEN BEHÄLTER
RÉCIPIENT MÉDICAL, ADAPTATEUR DE MONTAGE SUR LEDIT RÉCIPIENT MÉDICAL ET DISPOSITIF D'ADMINISTRATION DE MÉDICAMENTS COMPRENANT LEDIT RÉCIPIENT MÉDICAL

(43) Date of publication of application: 27.10.2021
(73) Proprietor: Becton Dickinson France, 38800 Le Pont-de-Claix (FR)
(72) Inventor: RIVIER, Cédric, 38340 Voreppe (FR)
(74) Representative: Germain Maureau

(56) References cited:
- EP-A1- 2 545 956
- WO-A1-2010/052517
- WO-A1-2012/049532

## Description

The present invention relates to a medical container, an adaptor for mounting onto said medical container, and a drug delivery device comprising said medical container.

In this application, the distal end of a component or of a device is to be understood as meaning the end furthest from the user's hand and the proximal end is to be understood as meaning the end closest to the user's hand. Likewise, in this application, the "distal direction" is to be understood as meaning the direction of injection, with respect to a medical container of the invention, and the "proximal direction" is to be understood as meaning the opposite direction to said direction of injection, that is to say the direction towards the user's hand holding a medical container as for an injection operation.

Basically, medical containers, such as for example syringes, may be made of a glass or plastic material. Preferably, the medical containers are made of glass for its high chemical passivity, its low gas permeability and high transparency, which allows an extended storage and an easy inspection.

The medical containers usually comprise a barrel forming a reservoir for containing a medical product. The barrel has a distal end in the form of a longitudinal tip defining an axial passageway through which the medical product is expelled from the container. However, this longitudinal tip does not allow parenteral administration by itself and must either comprise a staked needle or an adaptor allowing the connection of the syringe to a connector such as a needle hub or an intravenous (IV) line. This connector is typically screwed into the adaptor and engages the distal tip of the medical container to establish a fluid path between the reservoir and said connector.

It is known that the adaptor is strongly fixed to the longitudinal tip of the medical container in order to prevent accidental disengagement, either during connection of the connector onto the longitudinal tip, or caused by the fluid pressures within the drug delivery device and connector.

For example, the document WO2015/007650 discloses that adaptors may be secured around the longitudinal tip of the syringe by snap-fitting or friction force, for example by mechanical attaching means defined onto the longitudinal tip such as a groove or a ring.

The document WO2016/198580 alternatively discloses to provide adaptors with a mounting ring that is shaped and configured such that, when the adaptor is mounted around the longitudinal tip of the drug delivery device, a circumferential space is created between the mounting ring and the longitudinal tip, this circumferential space usually accommodating an adhesive layer in order efficiently to bond the adaptor to the longitudinal tip.

The document WO2010052517 further discloses an injection device and an adaptor allowing a safe connection of the adaptor on the injection device, in view of further connecting an IV (Intra Venous) connector to this injection device. The document EP2545956 discloses a drug delivery device having an end-piece and an adaptor for engaging around the end-piece so as to enable the safe connection of a connector on said end-piece. The document WO2012049532 discloses an adaptor intended to be used with a drug delivery device having an end-piece, the adaptor having a collar engageable around said-piece and enabling the safe connection of a connector on said collar.

It is however also important that the connector be properly screwed into the adaptor. Therefore, a user has to apply a sufficient torque when screwing the connector into the adaptor in order to get a proper fitting of the connector onto the distal tip of the medical container. Under-screwing or over-screwing of the connector into the adaptor may otherwise lead to a leakage or an ejection of the connector due to internal pressure when transferring a fluid between the container and the connector.

However, some connectors may abut against a distal face of the adaptor when being screwed into said adaptor. When such a contact happens, further rotation of the needle hub relative to the adaptor generates contact between the connector and the adaptor before the proper connection of the connector into the adaptor.

This early contact may give a user the false impression that a sufficient screwing torque is achieved, whereas the connector still does not properly fits onto the distal tip of the medical container. That is, if the user stops screwing, thinking that the connection is completed, the connector does not sufficiently engage the distal tip and a gap remains between the connector and an outer surface of this distal tip. Such a gap may lead to a leakage when transferring a fluid from the reservoir of the medical container to the connector.

There is therefore a need for a medical container preventing the risks of improper fitting and leakage between the connector and the distal tip even though the connector abuts against the distal face of the adaptor when being screwed into said adaptor.

An aspect of the invention is a medical container forming a reservoir for containing a medical product and having a longitudinal distal tip extending along a longitudinal axis A, the distal tip defining a passageway in fluid communication with said reservoir, wherein the distal tip includes
- a fitting portion configured to fit with a connector; and
- a mounting portion proximally located relative to said fitting portion and configured to slidably connect an adaptor to said distal tip,
characterized in that said mounting portion is configured to allow a free longitudinal movement of the adaptor relative to the distal tip when the adaptor is connected to the distal tip.

The medical container of the invention thus permits the adaptor to proximally slide along the distal tip if the connector abuts against a distal face of the adaptor. This prevents the screwing torque from increasing whereas the connector is not enough engaged onto the distal tip. Accordingly, a user will go on screwing the connector until this connector properly fits onto the distal tip of the medical container. As a result, risks of leakage are avoided.

In an embodiment, the mounting portion comprises guiding means configured to guide a translational movement of the adaptor relative to the distal tip and to prevent a rotation of the adaptor relative to the distal tip.

The translational movement is a longitudinal movement along the longitudinal axis A.

In an embodiment, the guiding means comprises at least one longitudinal bump, preferably three longitudinal bumps.

This allows a translational movement of the adaptor along the tip and blocks any rotation of the adaptor around said tip. The mounting portion of the distal tip, that may be made of a glass material or a plastic material, is indeed submitted to high stresses when the connector is secured to the adaptor. A single longitudinal bump is easier to manufacture. Three longitudinal bumps however provide an efficient guiding along the longitudinal direction A and an efficient blocking of any rotation around said longitudinal axis A.

In an embodiment, the mounting portion has a cylindrical shape.

The mounting portion thus guides the adaptor all along the mounting portion with reduced friction forces. The mounting portion has a constant outer diameter.

By cylindrical shape, it is meant that the mounting portion has the shape of any right cylinder, whose base may be a circle or a polygon, a square, an ellipse or any other non-circular geometrical shape. A mounting portion having a non-circular cross-section permits to guide the adaptor in the longitudinal direction while blocking a rotation of the adaptor around the distal tip. Such a mounting portion with a non-circular shape may form the guiding means in lieu of the at least one longitudinal bump. However, a mounting portion having a circular cross-section is easier to manufacture.

In an embodiment, the medical container comprises blocking means configured to limit the free longitudinal movement of the adaptor relative to the distal tip.

In an embodiment, the blocking means comprises a distal abutment surface and a proximal abutment surface.

The distal abutment surface prevents the removing of the adaptor from the distal tip of the medical container, and helps maintaining a closure cap of the medical container by blocking the adaptor in a distal-most position. The proximal abutment surface blocks the adaptor in a proximal-most position, thereby preventing the fact that the adaptor is too further engaged on the distal tip. In that case, it would not be possible anymore to engage the external thread of the connector into the inner thread of the adaptor.

In an embodiment, the distal abutment surface and the proximal abutment surface are respectively provided on a bump.

The adaptor is free to move relative to the distal tip along a predetermined amplitude a along the longitudinal axis A. By free longitudinal movement it is meant that the adaptor slides along the tip as soon as the adaptor is simply pushed proximally or distally in the axial direction. This predetermined amplitude a is comprised between 0.9mm and 1.3mm, preferably around 1.1 mm, along the longitudinal axis A. In other words, the blocking means may be configured so that the amplitude a of the adaptor free longitudinal movement is comprised between 0.9 mm and 1.3 mm, and is preferably around 1.1 mm. The greater the tip external diameter, the lower amplitude a needs to be. Conversely, the lower the tip external diameter, the greater amplitude a needs to be.

Another aspect of the invention is an adaptor for connecting the aforementioned medical container, said adaptor having connecting means configured to cooperate with complementary connecting means provided on a connector, and a mounting ring for mounting the adaptor onto the distal tip of the medical container, said mounting ring being configured to slidably engage the mounting portion of the distal tip.

In an embodiment, the mounting ring defines a through opening whose diameter is at least equal to or preferably greater than an outer diameter of the mounting portion of the distal tip.

There is thus no radial compression exerted by the mounting ring against the mounting portion of the distal tip.

Preferably, the difference between the diameter of the through opening and the outer diameter of the mounting portion is comprised between 0.1 mm and 0.3 mm, typically 0.2 mm.

In an embodiment, the mounting ring is in the form of a frustoconical disc.

The disc is distally inclined relative to a tubular wall of the adaptor. This reduces the insertion force while increasing the adaptor pull out force.

Another aspect of the invention is a drug delivery device comprising the aforementioned medical container and the aforementioned adaptor, wherein the adaptor is slidably mounted onto the mounting portion of the distal tip.

The adaptor is free to move relative to the distal tip along a predetermined amplitude, said predetermined amplitude being preferably comprised between 0.9mm and 1.3mm along the longitudinal axis A.

In an embodiment, the drug delivery device further includes a needle hub having an inner conduit configured to fit onto the distal tip and outer wings configured to engage an inner thread of the adaptor, said needle hub including a ramp for guiding a user's finger towards a pivoting arm in order to move the pivoting arm back towards a safety position wherein the pivoting arms covers a needle.

The invention and the advantages arising therefrom will clearly emerge from the detailed description that is given below with reference to the appended drawings as follows:
- Figure 1 is a perspective view of a medical container according to an embodiment of the invention,
- Figure 2 is a perspective view of an adaptor according to an embodiment of the invention,
- Figure 3 is a cross-section view of a drug delivery device according to an embodiment of the invention,
- Figures 4A and 4B are cross-section views of drug delivery devices according to an embodiment of the invention, when a needle hub is screwed into the adaptor,
- Figure 5 is a perspective view of a needle hub for cooperating with a drug delivery device according to an embodiment of the invention,
- Figure 6 is a cross section view of a needle hub for cooperating with a drug delivery device according to an embodiment of the invention.

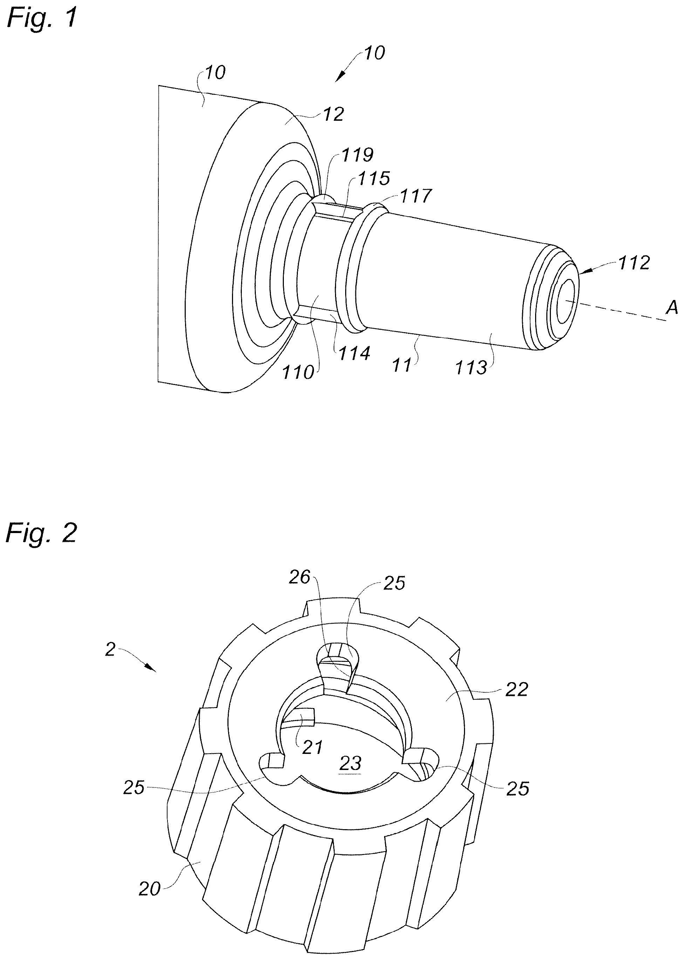

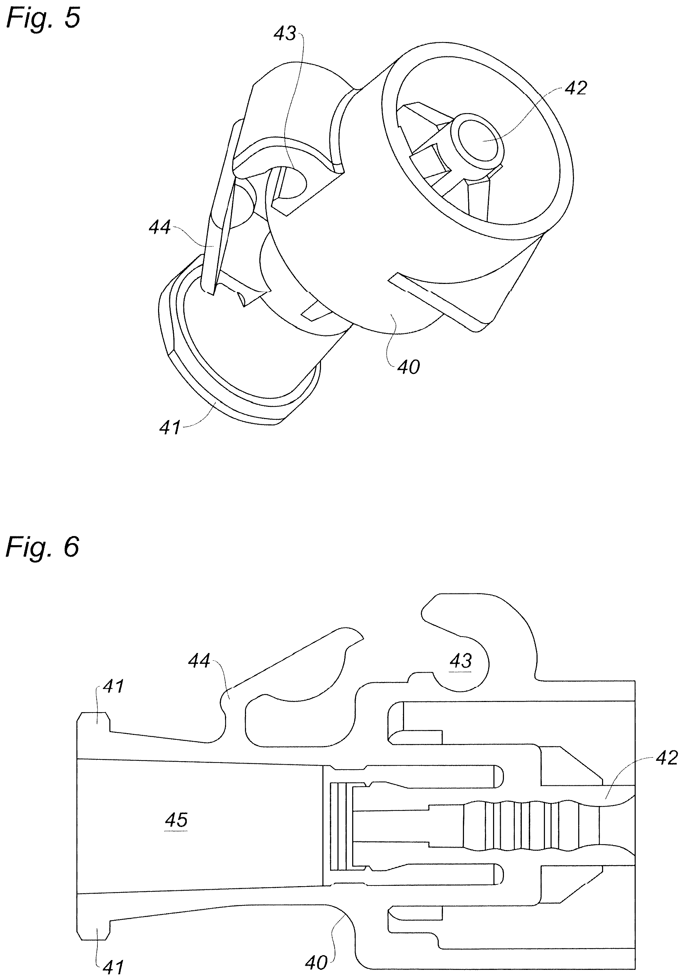

With reference to Figures 1 to 4B is shown a medical container 1, an adaptor 2 and a drug delivery device 3 according to an embodiment of the invention. The drug delivery device 3 is intended to cooperate with a connector such as a needle hub 40 as shown on Figures 5 and 6.

As illustrated on Figure 1, the medical container 1 comprises a barrel 10 defining a reservoir for containing a medical product. A distal tip 11 defining a passageway in fluid communication with the reservoir longitudinally extends along a longitudinal axis A of the medical container 1. The medical container 1 may be made of a glass material or a plastic material. The distal tip 11 may consequently be made either by glass forming or by injection molding.

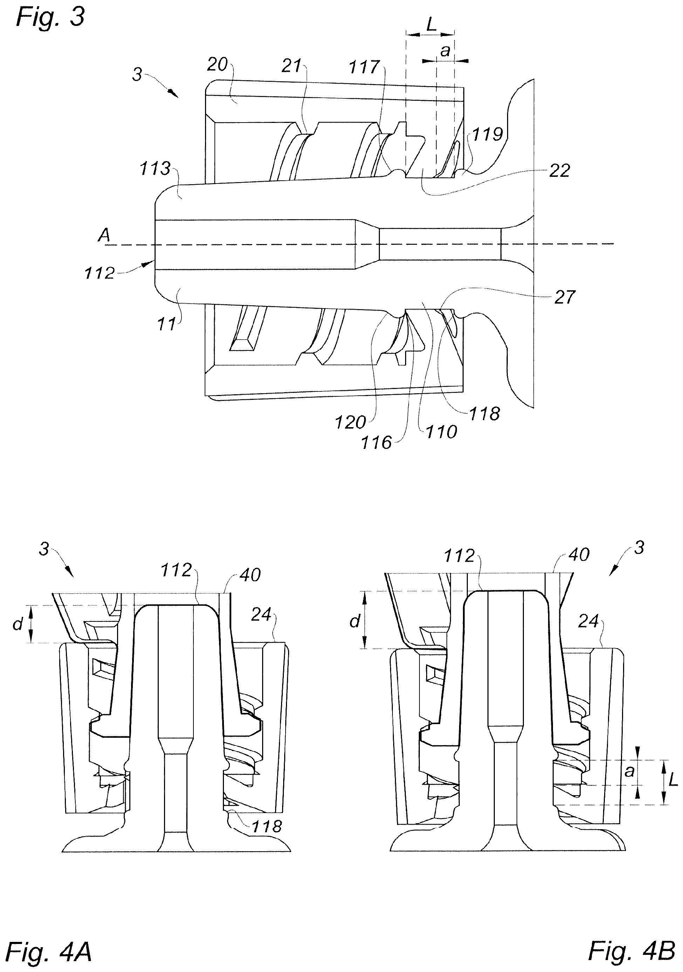

The distal tip 11 of the medical container 1 has a mounting portion 110. The mounting portion 110 is configured to receive a mounting ring 22 of the adaptor 2 for connecting the adaptor 2 onto the tip 11, as illustrated on Figure 3. The mounting portion 110 preferably extends in a proximal region of the distal tip 11. More specifically, the mounting portion 110 may be closer to a distal shoulder 12 of the barrel 10 than a distal face 112 of the longitudinal tip 11. Typically, the mounting portion 110 is disposed in the proximal-most third of the distal tip 11 if said distal tip 11 is divided into three equal thirds.

The distal tip 11 further comprises a fitting portion 113. The fitting portion 113 distally extends from the mounting portion 110. Said fitting portion 113 is configured to fit with an inner conduit of the needle hub 40 so as to establish a fluid path from the reservoir of the medical container 1 to a needle (not shown) of the needle hub 40. The fitting portion 113 preferably has a frustoconical shape.

According to the invention, the mounting portion 110 of the distal tip 11 comprises guiding means for guiding a longitudinal sliding movement of the adaptor 2 along the longitudinal axis A and for blocking a rotational movement of the adaptor 2 relative to the distal tip 11 around said longitudinal axis A.

As a result, the adaptor 2 is free to move relative to the distal tip 11 along a predetermined amplitude a. The predetermined amplitude a is defined by the difference between the length L of the mounting portion 110 and the thickness of the mounting ring 22, as illustrated in Figure 3. This predetermined amplitude a may be comprised between 0.9mm and 1.3mm, preferably around 1.1mm, along the longitudinal axis A. The adaptor 2 is free to slide along the longitudinal axis A within the predetermined amplitude a, i.e. between the distal-most position and the proximal-most position of the adaptor 2 when said adaptor 2 is mounted onto the distal tip 11.

This longitudinal movement of the adaptor 2 relative to the tip 11 permits to vary the distance d between the distal face 112 of the tip 11 and a distal face 24 of the adaptor 2 as visible on Figures 4A and 4B. As a result, the adaptor 2 may be pushed in a proximal direction, thereby increasing the distance d, by the needle hub 40 abutting against a distal face 24 of the adaptor 2. This allows the distal tip 11 to properly fit into an inner conduit of the needle hub 40. Risks of leakage due to an insufficient screwing of the needle hub 40 into the adaptor 2 because of a contact between the needle hub 40 and the distal face 24 of the adaptor 2 are thus avoided.

With reference to Figure 1, the guiding means may comprise at least one longitudinal bump 114. The least one longitudinal bump 114 extends in a straight direction parallel to the longitudinal axis A. Having a bump, that protrudes from an outer wall of the mounting portion 110, instead of a longitudinal groove that would form a recess with regard to said outer wall, has the advantage of strengthening, not weakening, said mounting portion 110 and said tip 11.

It should be noted that the outer diameter of the mounting portion 110, without bumps 114, 117, 119, is equal to or lower than, preferably equal to, the greatest outer diameter of the fitting portion 113 of the distal tip 11. In other words, the mounting portion 110 of the distal tip 11 is preferably no thinner than the rest of the tip 11. As a result, the mounting portion 110 is devoid of any groove or recess. This limits the risk of breakage, especially when the medical container 1 is made of glass.

As shown on Figure 1, the at least one longitudinal bump 114 may be in the form of an longitudinal rib extending all along the mounting portion 110.

The at least one longitudinal bump 114 may have a rounded shape, as illustrated on Figure 1. The at least one bump 114 may define recessed lateral edges 115.

Preferably, the guiding means may comprise three longitudinal bumps 114. Having three longitudinal bumps 114 offers a satisfactory repartition of the screwing torque around the tip 11 in order to avoid breakage without complicating the manufacturing process, especially when the tip 11 is made of glass. The three longitudinal bumps 114 may be regularly distributed around the mounting portion 110. The guiding means may however have less or more than three longitudinal bumps 114.

The mounting portion 110 preferably has cylindrical shape. Alternatively or complementarily to the at least one longitudinal bump 114, the guiding means may comprise the mounting portion 110 having a square, oval, polygonal or any other non-circular cross-section shape. Such a shape permits the adaptor 2 to slide along the mounting portion 110 without rotating around said mounting portion 110.

With reference to Figures 1 or 3 to 4B, the mounting portion 110 may further comprise blocking means for blocking the translational movement of the adaptor 2 relative to the tip 11.

The blocking means may comprise a first abutment surface 116 for blocking the adaptor 2 respectively in the distal direction. The first abutment surface 116 further prevents the pull out of the adaptor 2. In a distal-most position (Figure 3), the mounting ring 22 abuts against the first abutment surface 116.

The first abutment surface 116 defines the distal end of the mounting portion 110. The first abutment surface 116 may be arranged at the proximal side of a first orthoradial bump 117. This first orthoradial bump 117 protrudes from an outer wall of the distal tip 11. Having a first abutment surface 116, that is defined by a bump 117 instead of being defined by a shoulder. The first orthoradial bump 117 may be in the form of a circumferential rib extending all around the tip 11.

With reference to Figure 3, the first orthoradial bump 117 may have a ramp portion 120 provided on a distal side of said bump 117 in order to ease the passage of the mounting ring 22 of the adaptor 2 over said first orthorodial bump 117 in the proximal direction. This help the user mount the adaptor 2 onto the mounting portion 110 of the tip 11.

The blocking means may comprise a second abutment surface 118 for blocking the adaptor 2 respectively in the proximal direction. In a proximal-most position (Figure 4B), the mounting ring 22 abuts against the second abutment surface 118.

The second abutment surface 118 defines the proximal end of the mounting portion 110. The second abutment surface 118 may be arranged at the distal side of a second orthoradial bump 119. This second orthoradial bump 119 protrudes from an outer wall of the distal tip 11. Having a second abutment surface 118, that is defined by a bump 119 instead of being defined by a shoulder. The second orthoradial bump 119 may be in the form of a circumferential rib extending all around the tip 11.

The second abutment surface 118 may be located such that the distance d between the distal face of the tip 11 and the distal face 24 of the adaptor 2, when said adaptor 2 is connected to the tip 11, is no more than 2.7 mm. This allows the needle hub 40 to engage the adaptor 2, even though the adaptor 2 is in the proximal-most position. Indeed, if the distance d becomes too high, the needle hub 40 would otherwise not be able to engage the adaptor 2. More specifically, the maximum distance d of 2.7 mm allows the thread of the needle hub 40 to engage the thread 21 of the adaptor 2 when the distal tip 11 has a high external diameter.

The blocking means may define the length L of the mounting portion 110. With reference to Figure 3, this length L may be defined by the distance between the first and the second abutment surfaces 116, 118.

Figure 4A shows a needle hub 40 that is fitted to a distal tip 11 having a first diameter whereas Figure 4B shows a needle hub 40 that is fitted to a distal tip 11 having a second diameter that is smaller than said first diameter. As can be seen on these Figures, the sliding movement of the adaptor 2 along the distal tip 11 permits that the needle hub 40 reaches a predetermined position relative to the distal tip 11 wherein said needle hub 40 properly fits onto the tip 11. This predetermined position is advantageously defined as being the leakage limit of the needle hub 40 as defined in the ISO 80369-7 (2016) (paragraphs 6.1 and 6.2). Whether the needle hub 40 is under connected and has not reach said position, a leakage may occur. The sliding movement allows the needle hub 40 to reach the leakage limit position despite the contact that may occur between the needle and the adaptor 2 distal face.

With reference to Figures 2 and 3, the invention also relates to an adaptor 2 configured to be connected to the mounting portion 110 of the tip 11.

The adaptor 2 has a tubular wall 20 defining an inner cavity for receiving the needle hub 40. The inner cavity is provided with connecting means, such as an inner thread 21, for securing the needle hub 40 to the adaptor 2.

The adaptor 2 further has a mounting ring 22 inwardly protruding from a proximal end of the adaptor 2.. The mounting ring 22 allows connecting the adaptor 2 onto the distal tip 11.

The mounting ring 22 defines a through opening 23 for receiving the mounting portion 110 of the tip 11. The through opening 23 may be shaped to complementarily engage the mounting portion 110 of the tip 11. The thickness of the mounting ring 22 should be high enough to prevent the adaptor 2 pull out, but needs to be low enough to avoid increasing the length of the mounting portion 110 and thus the length of the distal tip 11. For instance, said thickness may be approximately comprised between 0.6 mm and 1.4 mm.

The width or diameter of said through opening 23 may be at least equal to, preferably greater than, the outer width or diameter of the mounting portion 110. As a result, a clearance is provided between the mounting ring 22 and the mounting portion 110. This favors the sliding movement of the adaptor 2 relative to the tip 11.

With reference to Figure 2, the mounting ring 22 comprises at least one guiding window 25. The at least one window 25 is configured to engage the at least one longitudinal bump 114 in order to guide the sliding movement of the adaptor 2 relative to the tip 11 and to block any rotation of the adaptor 2 relative to said tip 11. To that end, the at least one guiding window 25 and the longitudinal bump 114 may be complementarily shaped, while allowing the free sliding movement of the adaptor 2 relative to the distal tip 11. Preferably, the at least one guiding window 25 and the at least one longitudinal bump 114 are configured such that the adaptor 2 and the distal tip 11 are snap-fitted. For example, the at least one guiding window 25 may be slightly larger than the at least one longitudinal bump 114. The adaptor 2 may comprise as many guiding windows 25 as longitudinal bumps 114, for example three as illustrated on Figure 2.

As shown on Figure 2, the at least one guiding window 25 may open in the through opening 23 defined by said mounting ring 22. The adaptor 2 may thus be clipped onto the mounting ring 22 and the at least one longitudinal bump 114. The at least one window 25 has lateral edges 26 configured to engage the corresponding lateral edges 115 of the at least one longitudinal bump 114. This improves the clipping of the mounting ring 22 onto the at least one longitudinal bump 114. The lateral edges 26 may be part of snap-fitting means for connecting the adaptor 2 to the mounting portion 110.

With reference to Figure 3, the mounting ring 22 of the adaptor 2 may be in the form of a resilient frustoconical disc. The frustoconical is inclined relative to the longitudinal axis A and points towards the distal direction. This increases the adaptor 2 pull out force. This further helps the passage of the adaptor 2 over the first orthoradial bump 117. The inclined proximal side of the mounting ring 22 cooperates with the ramp portion 120 of said first orthoradial bump 117 to permit resilient deformation of the mounting ring 22. A beveled edge 27 may further be provided at the proximal side of the mounting ring 22.

The adaptor 2 may be made of any rigid polymer adapted to medical use, such as high density polyethylene (PE), polypropylene (PP), polycarbonate (PC), acrylonitrile butadiene styrene (ABS), polyoxymethylene (POM), polystyrene (PS), polybutylene terephthalate (PBT), polyamide (PA), and combinations thereof. To simplify its manufacturing, the adaptor 2 preferably consists of a single piece of material, preferably of a light-transmitting material. The adaptor 2 may be made by injection molding.

With reference to Figures 5 and 6, the invention further relates to a drug delivery device 3 comprising said medical container 1 and said adaptor 2 connected to the distal tip 11 of the medical container 1. The drug delivery device 3 may be a syringe, such as prefilled or pre-fillable syringe.

The drug delivery device 3 may comprise a connector such as a needle hub 40 as shown on Figures 5 and 6. The needle hub 40 comprises a proximal end defining an inner conduit 45 for receiving the distal tip 11 of the medical container 1. The distal end is provided with connecting means, such as two diametrically opposite outer wings 41, configured to engage the connecting means of the adaptor 2, such a the inner thread 21, in order to secure the needle hub 40 to the adaptor 2. The needle hub 40 further includes a distal end that may be provided with a mounting port 42 for mounting an injection needle and thereby establish a fluid path from the reservoir to the injection needle. A pivoting arm (not shown) may be connected at a pivot connection 43 in the form of a hook, said pivoting arm being configured to cover or unveil the injection needle. In order to help the user move the pivoting arm back towards the needle, the needle hub 40 may comprise a ramp 44 configured to guide a user's finger against the pivoting arm. For example, the needle hub 40 may be a BD Eclipse^{®}.

The invention thus permits a longitudinal movement of the adaptor 2 relative to the tip 11 of the medical container 1. This axial freedom allows a proper fitting between the needle hub 40 and the distal tip 11 of the medical container 1 in any situation. More specifically, the longitudinal movement of the adaptor 2 relative to the tip 11 permits the needle hub 40 to reach the leakage limit position even when the needle hub 40 abuts against the distal face of adaptor 2. The longitudinal movement of the adaptor 2 relative to the tip 11 permits the needle hub 40 to reach the leakage limit position regardless of the distal tip 11 diameter. As a result, risks of leakage due to unproper fitting between the tip 11 and the needle hub 40 are avoided.

## Claims

1. A medical container (1) forming a reservoir for containing a medical product and having a longitudinal distal tip (11) extending along a longitudinal axis (A), the distal tip (11) defining a passageway in fluid communication with said reservoir, wherein the distal tip (11) includes
- a fitting portion (113) configured to fit with a connector; and
- a mounting portion (110) proximally located relative to said fitting portion (113) and configured to slidably connect an adaptor (2) to said distal tip (11),
wherein said mounting portion (110) is configured to allow a free longitudinal movement of the adaptor (2) relative to the distal tip (11) when the adaptor (2) is connected to the distal tip (11),
wherein the mounting portion (110) comprises guiding means configured to guide a translational movement of the adaptor (2) relative to the distal tip (11) and to prevent a rotation of the adaptor (2) relative to the distal tip (11),
the guiding means comprising at least one longitudinal bump (114), preferably three longitudinal bumps (114), configured to engage at least one complementarily shaped guiding window (25) of a mounting ring (22) of the adaptor (2), and **characterized in that**
the least one longitudinal bump (114) has a rounded shape and defines recessed lateral edges (115) configured to receive lateral edges (26) of said at least one guiding window (25), thereby allowing a snap-fit connection between the adaptor (2) and the mounting portion (110) of the distal tip (11).

2. The medical container (1) according to the preceding claim, wherein the mounting portion (110) has a cylindrical shape.

3. The medical container (1) according to any of the preceding claims, comprising blocking means configured to limit the free longitudinal movement of the adaptor (2) relative to the distal tip (11).

4. The medical container (1) according to the preceding claim, wherein the blocking means comprises a distal abutment surface (116) and a proximal abutment surface (118).

5. The medical container (1) according to the preceding claim, wherein the distal abutment surface (116) and the proximal abutment surface (118) are respectively provided on a bump (117, 119).

6. The medical container (1) according to any of claims 3-5, wherein the blocking means are configured so that an amplitude (a) of the adaptor (2) free longitudinal movement is comprised between 0.9 mm and 1.3 mm, and is preferably around 1.1 mm.

7. An adaptor (2) for connecting the medical container (1) according to any of the preceding claims, said adaptor (2) having connecting means configured to cooperate with complementary connecting means provided on a connector, and a mounting ring (22) for mounting the adaptor (2) onto the distal tip (11) of the medical container (1), said mounting ring (22) being configured to slidably engage the mounting portion (110) of the distal tip (11), wherein the mounting ring (22) comprises at least one guiding window (25) configured to engage an at least one complementarily shaped longitudinal bump (114) of said mounting portion (110) in order to guide the sliding movement of the adaptor (2) relative to the tip (11) and to block any rotation of the adaptor (2) relative to said tip (11), and **characterized in that** this at least one window (25) has lateral edges (26) configured to engage corresponding lateral edges (115) of the at least one longitudinal bump (114) having a rounded shape, said lateral edges (26) forming snap-fitting means for connecting the adaptor (2) to the mounting portion (110).

8. The adaptor (2) according to the preceding claim, wherein the mounting ring (22) defines a through opening (23) whose diameter is at least equal to or preferably greater than an outer diameter of the mounting portion (110) of the distal tip (11).

9. The adaptor (2) according to any of claims 7-8, wherein the mounting ring (22) is in the form of a frustoconical disc.

10. A drug delivery device (3) comprising the medical container (1) according to any of claims 1-6 and the adaptor (2) according to any of claims 7-9, wherein the adaptor (2) is slidably mounted onto the mounting portion (110) of the distal tip (11).

11. The drug delivery device (3) according to the preceding claim, wherein the drug delivery device (3) further includes a needle hub (40) having an inner conduit configured to fit onto the distal tip (11) and outer wings (41) configured to engage an inner thread (21) of the adaptor (2), said needle hub (40) including a ramp (44) for guiding a user's finger towards a pivoting arm in order to move the pivoting arm back towards a safety position wherein the pivoting arms covers a needle.

## Patentansprüche

1. Medizinischer Behälter (1), der ein Reservoir zum Enthalten eines medizinischen Produkts bildet und eine distale Längsspitze (11) aufweist, die sich entlang einer Längsachse (A) erstreckt, wobei die distale Spitze (11) einen Durchgang in strömungstechnischer Kommunikation mit dem Reservoir definiert, wobei die distale Spitze (11) beinhaltet
- einen Passabschnitt (113), der so konfiguriert ist, dass er mit einem Verbindunger zusammenpasst; und
- einen Montageabschnitt (110), der sich proximal relativ zum Passabschnitt (113) befindet und so konfiguriert ist, dass er einen Adapter (2) gleitend mit der distalen Spitze (11) verbindet,
wobei der Montageabschnitt (110) so konfiguriert ist, dass er eine freie Längsbewegung des Adapters (2) relativ zur distalen Spitze (11) ermöglicht, wenn der Adapter (2) mit der distalen Spitze (11) verbunden ist,
wobei der Montageabschnitt (110) Führungsmittel umfasst, die so konfiguriert sind, dass sie eine Verschiebebewegung des Adapters (2) relativ zur distalen Spitze (11) führen und eine Drehung des Adapters (2) relativ zur distalen Spitze (11) verhindern,
wobei die Führungsmittel mindestens eine Längserhebung (114), vorzugsweise drei Längserhebungen (114), umfassen, die so konfiguriert sind, dass sie mit mindestens einem komplementär geformten Führungsfenster (25) eines Montagerings (22) des Adapters (2) in Eingriff gelangen, und
**dadurch gekennzeichnet, dass**
die mindestens eine Längserhebung (114) eine abgerundete Form aufweist und vertiefte Seitenkanten (115) definiert, die so konfiguriert sind, dass sie Seitenkanten (26) des mindestens einen Führungsfensters (25) aufnehmen, wodurch eine Rastverbindung zwischen dem Adapter (2) und dem Montageabschnitt (110) der distalen Spitze (11) ermöglicht wird.

2. Medizinischer Behälter (1) nach dem vorstehenden Anspruch, wobei der Montageabschnitt (110) eine zylindrische Form aufweist.

3. Medizinischer Behälter (1) nach einem der vorstehenden Ansprüche, der Blockiermittel umfasst, die so konfiguriert sind, dass sie die freie Längsbewegung des Adapters (2) relativ zur distalen Spitze (11) begrenzen.

4. Medizinischer Behälter (1) nach dem vorstehenden Anspruch, wobei die Blockiermittel eine distale Anschlagfläche (116) und eine proximale Anschlagfläche (118) umfassen.

5. Medizinischer Behälter (1) nach dem vorstehenden Anspruch, wobei die distale Anschlagfläche (116) und die proximale Anschlagfläche (118) jeweils an einer Erhebung (117, 119) vorgesehen sind.

6. Medizinischer Behälter (1) nach einem der Ansprüche 3-5, wobei die Blockiermittel so konfiguriert sind, dass eine Amplitude (a) der freien Längsbewegung des Adapters (2) im Bereich zwischen 0,9 mm und 1,3 mm liegt und vorzugsweise etwa 1,1 mm beträgt.

7. Adapter (2) zum Verbinden des medizinischen Behälters (1) nach einem der vorstehenden Ansprüche, wobei der Adapter (2) Verbindungsmittel, die so konfiguriert sind, dass sie mit komplementären Verbindungsmitteln zusammenwirken, die an einem Verbinder vorgesehen sind, und einen Montagering (22) zum Montieren des Adapters (2) an der distalen Spitze (11) des medizinischen Behälters (1) aufweist, wobei der Montagering (22) so konfiguriert ist, dass er gleitend mit dem Montageabschnitt (110) der distalen Spitze (11) in Eingriff gelangt, wobei der Montagering (22) mindestens ein Führungsfenster (25) umfasst, das so konfiguriert ist, dass es mit mindestens einer komplementär geformten Längserhebung (114) des Montageabschnitts (110) in Eingriff gelangt, um die Gleitbewegung des Adapters (2) relativ zur Spitze (11) zu führen und eine Drehung des Adapters (2) relativ zur Spitze (11) zu blockieren, und **dadurch gekennzeichnet, dass** dieses mindestens
eine Fenster (25) Seitenkanten (26) aufweist, die so konfiguriert sind, dass sie mit entsprechenden Seitenkanten (115) der mindestens einen Längserhebung (114), die eine abgerundete Form aufweist, in Eingriff gelangen, wobei die Seitenkanten (26) Verrastungsmittel zum Verbinden des Adapters (2) mit dem Montageabschnitt (110) bilden.

8. Adapter (2) nach dem vorstehenden Anspruch, wobei der Montagering (22) eine Durchgangsöffnung (23) definiert, deren Durchmesser mindestens gleich oder vorzugsweise größer ist als ein Außendurchmesser des Montageabschnitts (110) der distalen Spitze (11).

9. Adapter (2) nach einem der Ansprüche 7-8, wobei der Montagering (22) in Form einer kegelstumpfförmigen Scheibe vorliegt.

10. Arzneimittelabgabevorrichtung (3), die den medizinischen Behälter (1) nach einem der Ansprüche 1-6 und den Adapter (2) nach einem der Ansprüche 7-9 umfasst, wobei der Adapter (2) gleitend auf dem Montageabschnitt (110) der distalen Spitze (11) montiert ist.

11. Arzneimittelabgabevorrichtung (3) nach dem vorstehenden Anspruch, wobei die Arzneimittelabgabevorrichtung (3) weiter einen Nadelansatz (40) beinhaltet, der einen inneren Kanal, der so konfiguriert ist, dass er auf die distale Spitze (11) passt, und äußere Flügel (41) aufweist, die so konfiguriert sind, dass sie mit einem Innengewinde (21) des Adapters (2) in Eingriff gelangen, wobei der Nadelansatz (40) eine Rampe (44) zum Führen des Fingers eines Benutzers in Richtung eines Schwenkarms beinhaltet, um den Schwenkarm zurück in Richtung einer Sicherheitsposition zu bewegen, in der die Schwenkarme eine Nadel abdecken.

## Revendications

1. Récipient médical (1) formant un réservoir pour contenir un produit médical et ayant une pointe distale longitudinale (11) s'étendant le long d'un axe longitudinal (A), la pointe distale (11) définissant un passage en communication fluidique avec ledit réservoir, dans lequel la pointe distale (11) comprend
- une partie d'ajustement (113) configurée pour s'ajuster à un connecteur ; et
- une partie de montage (110) située de manière proximale par rapport à ladite partie d'ajustement (113) et configurée pour relier en coulissement un adaptateur (2) à ladite pointe distale (11),
dans lequel ladite partie de montage (110) est configurée pour permettre un mouvement longitudinal libre de l'adaptateur (2) par rapport à la pointe distale (11) lorsque l'adaptateur (2) est relié à la pointe distale (11),
dans lequel la partie de montage (110) comprend des moyens de guidage configurés pour guider un mouvement de translation de l'adaptateur (2) par rapport à la pointe distale (11) et pour empêcher une rotation de l'adaptateur (2) par rapport à la pointe distale (11),
les moyens de guidage comprenant au moins une bosse longitudinale (114), de préférence trois bosses longitudinales (114), configurée pour s'engager dans au moins une fenêtre de guidage de forme complémentaire (25) d'une bague de montage (22) de l'adaptateur (2), et
**caractérisé en ce que**
l'au moins une bosse longitudinale (114) a une forme arrondie et définit des bords latéraux en retrait (115) configurés pour recevoir les bords latéraux (26) de ladite au moins une fenêtre de guidage (25), permettant ainsi une connexion par encliquetage entre l'adaptateur (2) et la partie de montage (110) de la pointe distale (11).

2. Récipient médical (1) selon la revendication précédente, dans lequel la partie de montage (110) a une forme cylindrique.

3. Récipient médical (1) selon l'une des revendications précédentes, comprenant des moyens de blocage configurés pour limiter le mouvement longitudinal libre de l'adaptateur (2) par rapport à la pointe distale (11).

4. Récipient médical (1) selon la revendication précédente, dans lequel le moyen de blocage comprend une surface de butée distale (116) et une surface de butée proximale (118).

5. Récipient médical (1) selon la revendication précédente, dans lequel la surface de butée distale (116) et la surface de butée proximale (118) sont respectivement prévues sur une bosse (117, 119).

6. Récipient médical (1) selon l'une des revendications 3 à 5, dans lequel les moyens de blocage sont configurés de sorte qu'une amplitude (a) du mouvement longitudinal libre de l'adaptateur (2) soit comprise entre 0,9 mm et 1,3 mm, et soit de préférence d'environ 1,1 mm.

7. Adaptateur (2) pour relier le récipient médical (1) selon l'une des revendications précédentes, ledit adaptateur (2) ayant des moyens de connexion configurés pour coopérer avec des moyens de connexion complémentaires prévus sur un connecteur, et une bague de montage (22) pour monter l'adaptateur (2) sur la pointe distale (11) du récipient médical (1), ladite bague de montage (22) étant configurée pour s'engager en coulissement avec la partie de montage (110) de la pointe distale (11), dans lequel la bague de montage (22) comprend au moins une fenêtre de guidage (25) configurée pour s'engager avec au moins une bosse longitudinale de forme complémentaire (114) de ladite partie de montage (110) afin de guider le mouvement de coulissement de l'adaptateur (2) par rapport à la pointe (11) et de bloquer toute rotation de l'adaptateur (2) par rapport à ladite pointe (11), et **caractérisé en ce que** cette au moins une fenêtre (25) a des bords latéraux (26) configurés pour s'engager avec des bords latéraux correspondants (115) de l'au moins une bosse longitudinale (114) ayant une forme arrondie, lesdits bords latéraux (26) formant des moyens d'encliquetage pour relier l'adaptateur (2) à la partie de montage (110).

8. Adaptateur (2) selon la revendication précédente, dans lequel la bague de montage (22) définit une ouverture traversante (23) dont le diamètre est au moins égal, ou de préférence supérieur, au diamètre extérieur de la partie de montage (110) de la pointe distale (11).

9. Adaptateur (2) selon l'une des revendications 7 et 8, dans lequel la bague de montage (22) a la forme d'un disque tronconique.

10. Dispositif d'administration de médicament (3) comprenant le récipient médical (1) selon l'une des revendications 1 à 6 et l'adaptateur (2) selon l'une des revendications 7 à 9, dans lequel l'adaptateur (2) est monté en coulissement sur la partie de montage (110) de la pointe distale (11).

11. Dispositif d'administration de médicament (3) selon la revendication précédente, dans lequel le dispositif d'administration de médicament (3) comprend en outre un raccord d'aiguille (40) ayant un conduit interne configuré pour s'ajuster sur la pointe distale (11) et des ailes externes (41) configurées pour s'engager avec un filetage interne (21) de l'adaptateur (2), ledit raccord d'aiguille (40) comprenant une rampe (44) pour guider le doigt d'un utilisateur vers un bras pivotant afin de ramener le bras pivotant vers une position de sécurité dans laquelle le bras pivotant couvre une aiguille.
